# NOUVEAU FASCICULE DE BREVET EUROPEEN

(11) **EP 0 665 008 B2**
(45) Date de publication et mention de la décision concernant l'opposition: **17.03.2004**
(45) Mention de la délivrance du brevet: 11.06.1997
(21) Numéro de dépôt: 95400156.6
(22) Date de dépôt: 25.01.1995
(51) Int. Cl.: A61K 7/48, A61K 7/027

(54) **Procédé de préparation de Compositions cosmétiques ou dermatologiques anhydres contenant l'association d'une huile de silicone et d'une cire d'un homo- ou copolymère d'éthylène**
Wasserfreies kosmetisches oder dermatologisches Präparat das eine Gemisch eines Silikonöles und eines Wachses des Homo- oder Copolymeres Äthylens enthält
Anhydrous cosmetic or dermatological composition containing an association of silicone oil and an ethylenic homo- or copolymeric wax

(30) Priorité: 26.01.1994 FR 9400843
(43) Date de publication de la demande: 02.08.1995
(73) Titulaire: L'OREAL, 75008 Paris (FR)
(72) Inventeur: Arnaud, Pascal, F-94000 Creteil (FR); Mellul Myriam, F-94240 L'Hay les Roses (FR)
(74) Mandataire: Le Coupanec, Pascale

(56) Documents cités:
- EP-A- 0 205 961
- EP-A- 0 385 312
- EP-A- 0 511 092

## Description

La présente invention a pour objet un procédé de préparation d'une composition cosmétique ou dermatologique, anhydre, contenant dans sa phase grasse l'association d'une huile de silicone et d'une cire d'un homopolymère ou copolymère d'éthylène.

L'utilisation d'huiles de silicone dans la formulation de produits pour une application topique est particulièrement recherchée dans la mesure où celles-ci présentent à la tcis une innocuité, une inertie chimique et de très bonnes propriétés lubrifiantes et filmogènes. En particulier, par application sur la peau ou sur les fibres kératiniques, elles conduisent à la formation d'un film présentant à la fois une homogénéité, une douceur et une brillance particulièrement satisfaisantes.

Les compositions anhydres à application topique sont généralement des compositions solides ou pâteuses nécessitant pour ce faire la présence de cires telles que notamment des cires naturelles ou des cires de paraffine.

Toutefois, lorsque les proportions en huile de silicone et en cire dépassent respectivement 5 % et 3 % en poids, on a constaté une mauvaise compatibilité, de telle sorte que l'on ne peut obtenir un mélange homogène, après refroidissement, que pour des intervalles de proportions prédéterminés particulièrement limités. On entend par mélange homogène, un mélange dans lequel les différents constituants sont répartis de façon identique en tous points du mélange. L'absence de compatibilité des constituants d'une composition se traduit par une dégradation de celle-ci et notamment par l'apparition d'une synérese. On a en outre constate que ce problème de compatibilité des huiles de silicone intervenait avec la plupart des cires.

Diverses solutions ont été envisagées pour résoudre ce problème. Ainsi, il a été décrit dans GB 1.140.536, l'utilisation de cires comprenant au moins 15 % de cire de silicone. Il a également été envisagé dans EP-A-205.961, l'utilisation de cires de paraffine, de cires microcristallines ou hydrocarbonées en association avec une résine et une polyoléfine. Il a également été décrit dans US 5.085.855, l'utilisation d'une combinaison d'huile de lanoline, de cire de lanoline, d'agents gélifiants et de polymères hydrocarbonés. Toutefois, selon ces diverses solutions, il est nécessaire de combiner les divers éléments dans des proportions prédéterminées limitées, et toute addition d'un composé supplémentaire nécessite l'établissement préalable d'un diagramme de compatibilité d'autant plus complexe que le nombre de composés formant la composition est élevé.

Après de nombreuses recherches, on a maintenant constaté de façon surprenante et inattendue qu'en associant une huile de silicone particulière et une cire d'un homopolymère d'éthylène ou d'un copolymère d'éthylène résultant d'une sélection portant à la fois sur le point de fusion de la cire mais également sur sa masse moléculaire, on pouvait obtenir des compositions cosmétiques anhydres à base d'huile de silicone, homogènes, sans pour autant être limité par un intervalle de proportion restreint.

La présente invention a donc pour objet un procédé de préparation d'une composition cosmétique ou dermatologique anhydre ayant une phase grasse contenant l'association d'une huile de silicone et d'une cire, dans lequel, afin d'obtenir une phase grasse homogène, on mélange à chaud :
i) de 5 à 97 % en poids par rapport au poids total de ladite phase grasse, d'au moins une huile de silicone répondant à la formule suivante : 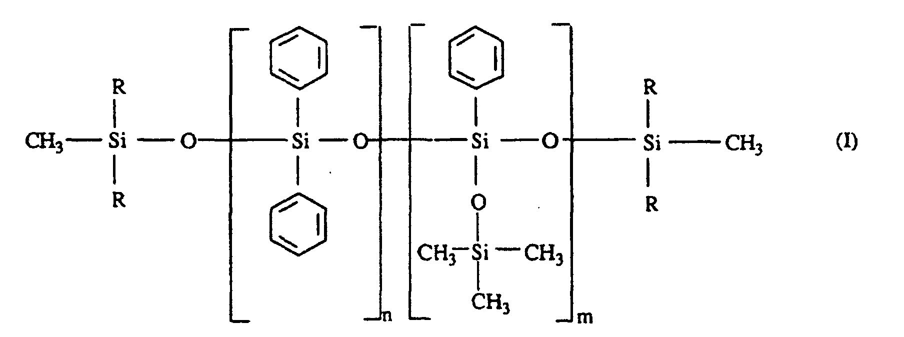 dans laquelle :
   R représente un radical alkyle comportant 1 à 30 atomes de carbone, un radical aryle ou aralkyle,
   n représente un nombre entier compris entre 0 et 100, et
   m représente un nombre entier compris entre 0 et 100,
   sous réserve que la somme n + m soit comprise entre 1 et 100, et
ii) de 3 à 50 % en poids par rapport au poids total de ladite phase grasse, d'une cire ayant un point de fusion compris entre 50 et 135°C, et étant constituée d'au moins un polymère de poids moléculaire compris entre 200 et 1.500 choisi parmi les homopolymères d'éthylène et les copolymères d'éthylène et d'un monomère répondant à la formule :

   CH₂= CH-R' (II)

   dans laquelle :
   R' représente un radical alkyle ayant de 1 à 30 atomes de carbone, un radical aryle ou aralkyle.

Parmi les radicaux alkyles ayant de 1 à 30 atomes de carbone, on peut citer les radicaux méthyle, éthyle, propyle, isopropyle, décyle, dodécyle et octadécyle.

Le radical aryle est de préférence le radical phényle ou tolyle.

Le radical aralkyle est de préférence le radical benzyle ou phénéthyle.

Parmi les huiles de silicone répondant à la formule (I), on utilise de préférence celles présentant une viscosité, mesurée à 25°C, comprise entre 5 · 10⁻⁶ et 500 · 10⁻⁶ m²/s (5 et 500 centistockes (cSt)).

Parmi celles-ci, on peut citer notamment celle commercialisée sous la dénomination de "Abil AV 8853" par la Société Goldschmidt, celles commercialisées sous les dénominations de "DC 556" et "SP 558" par la Société Dow Corning et celle commercialisée sous la dénomination de "Silbione 70633.V 30" par la Société Rhône Poulenc.

Selon un mode de réalisation préféré, l'huile de silicone de formule (I) est présente en une proportion comprise entre 10 et 90 % en poids par rapport au poids total de la phase grasse.

L'utilisation des cires d'homo- ou de copolymères d'éthylène telles que définies précédemment confère au mélange de très nombreux avantages. En effet, le mélange obtenu présente une grande stabilité thermique, un caractère thixotrope conduisant à d'excellentes propriétés d'étalement, une très bonne résistance à l'eau permettant une bonne tenue cosmétique des produits sur la peau, ainsi que sur les fibres kératiniques. Par ailleurs, ces cires permettent de disperser une importante quantité de particules solides tout en conservant de bonnes propriétés d'application. De plus, étant des produits de synthèse, elles ne présentent pas les problèmes de variabilité observée avec les composés naturels.

Selon un mode de réalisation préféré, la cire utilisée telle que définie précédemment est choisie parmi les homopolymères d'éthylène, les copolymères éthylène-propylène et les copolymères éthylène-hexène.

Parmi les homopolymères d'éthylène utilisables selon l'invention, on peut citer notamment ceux commercialisés sous les dénominations de "Polywax 500", "Polywax 655" et "Polywax 1.000" par la Société Bareco, ceux commercialisés sous les dénominations de "PE 1.500 F" et "PEW 1.555" par la Société Langer & Co., celui commercialisé sous la dénomination de "TN WAX 1.495" par la Société R.T.Newey et "AC 1702" par la Société Allied Chemical Corp..

Parmi les copolymères d'éthylène utilisables selon l'invention, on peut citer notamment les copolymères éthylène-propylène commercialisés sous les dénominations de "Petrolite CP-7" et "Petrolite CP-12" par la Société Bareco et les copolymères éthylène-hexène commercialisés sous les dénominations de "Petrolite CH-7" et "Petrolite CH-12" par la Société Bareco.

Selon un mode de réalisation préféré, la cire utilisée telle que définie précédemment est présente en une proportion comprise entre 5 et 30% en poids par rapport au poids total de la phase grasse.

Le mélange d'huile de silicone et de cire d'un homo- ou copolymère d'éthylène tel que défini ci-dessus est généralement présent dans la composition selon l'invention en une proportion comprise entre 3 et 100 % en poids par rapport au poids total de la composition.

En plus des deux constituants du mélange tel que défini ci-dessus, la phase grasse peut en outre comprendre des additifs ou corps gras choisis parmi les huiles et/ou les cires. La proportion en additif ou corps gras présent dans la composition est généralement comprise entre 0,5 et 92 % en poids par rapport au poids total de la phase grasse et de préférence entre 2 et 85 %.

Contrairement aux compositions connues, pour lesquelles il est nécessaire d'établir des diagrammes de compatibilité complexe, c'est-à-dire prenant en compte tous les éléments présents dans la composition, il suffit pour obtenir des compositions conformément à l'invention, d'établir un diagramme de compatibilité de l'additif avec l'un des composés de l'association, c'est-à-dire soit avec la cire d'homo- ou copolymère d'éthylène, soit avec l'huile de silicone. Si l'additif est compatible avec l'un d'eux, il est nécessairement compatible avec leur association.

Les huiles éventuellement présentes dans la phase grasse peuvent être d'origine minérale, animale, végétale ou de synthèse.

Comme huile d'origine minérale, on peut citer notamment l'huile de paraffine, l'huile de vaseline et les huiles minérales en général ayant un point d'ébullition compris entre 310 et 410°C.

Comme huile d'origine animale, on peut citer notamment le perhydrosqualène.

Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germe de blé.

Comme huile de synthèse, on peut citer notamment les esters de synthèse tels que l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléoate de décyle, le laurate d'hexyle, le dicaprylate de propylène glycol et l'adipate de diisopropyle.

Comme huiles également utilisables, on peut citer les alcools organiques tels que l'alcool oléique, l'alcool linoléique, l'alcool linolénique, l'alcool isostéarylique et l'octyldodécanol et les esters dérivés d'acide lanolique tels que le lanolate d'isopropyle et le lanolate d'isocétyle.

On peut encore citer les acétylglycérides, les octanoates et décanoates d'alcools et de polyalcools tels que ceux de glycol et glycérol et les ricinoléates d'alcools et de polyalcools tels que ceux de cétyle.

Les cires éventuellement présentes dans la phase grasse peuvent être d'origine minérale, fossile, animale, végétale ou bien encore être des huiles hydrogénées ou des esters gras concrets à 25°C.

Comme cires minérales utilisables, on peut citer notamment les cires microcristallines, la paraffine, la vaseline et la cérésine.

Comme cires fossiles, on peut citer l'ozokérite et la cire de montan.

Comme cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.

Comme cires d'origine végétale, on peut citer notamment la cire de candellila, la cire de carnauba, la cire du Japon et le beurre de cacao.

Comme huiles hydrogénées concrètes à 25°C, on peut citer notamment l'huile de ricin hydrogénée, l'huile de palme hydrogénée, le suif hydrogéné et l'huile de coco hydrogénée.

Comme esters gras concrets à 25°C, on peut citer notamment le mono-myristate de propylène glycol et le myristate de myristyle.

Comme cires, on peut encore citer l'alcool cétylique, l'alcool stéarylique, les mono-, di- et triglyglycérides concrets à 25°C, le monoéthanolamide stéarique, la colophane et ses dérivés tels que les ablétates de glycol et de glycérol, les sucro-glycérides et les oléates, myristates, lanolates, stéarates et dihydroxystéarates de calcium, de magnésium, de zinc et d'aluminium.

La proportion en cire telle que définie ci-dessus est de préférence inférieure ou égale à celle des cires d'homo- ou de copolymères d'éthylène.

On peut en outre introduire dans la phase grasse des compositions des agents gélifiants huileux.

Comme agents gélifiants huileux, on peut citer notamment les esters métalliques tels que le stéarate de polyoxyaluminium et l'hydroxystéarate d'aluminium ou de magnésium, les dérivés de cholestérol et en particulier l'hydroxycholestérol et les minéraux argileux gonflants en présence d'huile et notamment ceux appartenant au groupe des montmorillonites.

Selon un mode de réalisation particulier, les compositions obtenues selon l'invention peuvent en outre contenir des charges, c'est-à-dire des composés solides sous forme de poudre. La proportion en charges dans les compositions est généralement comprise entre 0,5 et 97 % en poids et de préférence entre 1 et 40 % en poids par rapport au poids total de la composition.

Les composés sous forme de poudre utilisables peuvent être d'origine naturelle ou synthétique. Parmi celles-ci, on peut citer notamment :
(a) les poudres minérales, telles que le talc, le kaolin, le mica, la silice, les silicates, l'alumine, les zéolites, l'hydroxyapatite, la séricite, le dioxyde de titane, les micatitanes, l'oxyde de zinc, le sulfate de baryum, les oxydes de fer, le violet de manganèse, l'oxyde de chrome, le bleu d'outre-mer, l'oxychlorure de bismuth, le nitrure de bore et les poudres métalliques telles que la poudre d'aluminium.
(b) les poudres végétales, telles que les poudres d'arnidon de maïs, de froment ou de riz.
(c) les poudres organiques, telles que les poudres de nylon, de polyamide, de polyester, de polytétrafluoroéthylène ou de polyéthylène.
(d) les poudres organo-métalliques, telles que les pigments, associant le zirconium, le baryum ou l'aluminium à des colorants organiques.

Les poudres décrites précédemment peuvent en outre être enrobées par exemple par des sels métalliques d'acides gras, des acides aminés, de la lécithine, du collagène, des composés siliconés, des composés fluorés, des composés fluorosiliconés ou par tout autre enrobage usuel.

Les compositions obtenues selon l'invention peuvent comprendre en outre un additif lipophile choisi parmi les agents tensio-actifs, les filtres, les vitamines, les hormones, les agents anti-oxydants, les conservateurs, les colorants, les parfums et leurs mélanges.

Les compositions cosmétiques ou dermatologiques obtenues selon l'invention peuvent se présenter sous différentes formes telles que sous forme de gels huileux, de produits solides tels que des poudres compactées ou bien encore de sticks. Elles peuvent être utilisées notamment comme produits de soin, de nettoyage ou de maquillage.

Lorsqu'elles se présentent sous forme de produits de maquillage, elles peuvent être en particulier des fonds de teint, des mascaras, des rouges à lèvres, des fards à paupières ou à joues.

Les compositions sont préparées selon les méthodes conventionnelles, c'est-à-dire par homogénéisation à chaud de leurs différents constituants puis refroidissement.

Les différentes formes de compositions décrites précédemment sont obtenues selon la nature et les proportions des composés introduits ainsi que selon le mode de refroidissement utilisé. Ainsi, par simple refroidissement en présence ou non d'agitation, on peut obtenir une composition sous forme de gel. On peut également obtenir des compositions de différentes formes par coulage du mélange à chaud dans différents types de conditionnement.

Les produits sous forme compactée sont obtenus bien entendu par application d'une pression sur le produit.

On va maintenant donner à titre d'illustration, plusieurs exemples de réalisation de compositions conformément à l'invention.

### EXEMPLE 1 : Gel huileux

| *Phase A :* | |
|---|---|
| - Huile de silicone commercialisée sous la dénomination de "SF-558" par la Société Dow Corning | 55 g |
| - Huile de sésame | 20 g |

| *Phase B :* | |
|---|---|
| - Homopolymère d'éthylène commercialisé sous la dénomination de "AC 1702" par la Société Allied Chemical | 20 g |
| - Homopolymère d'éthylène commercialisé sous la dénomination de "Polywax 655" par la Société Bareco | 5 g |

On procède à la préparation de la phase A par mélange de ses constituants sous agitation à température ambiante. Après homogénéisation, on ajoute alors les constituants de la phase B, puis chauffe à une température d'environ 107°C. Après fusion et homogénéisation, on obtient un milieu limpide. Le gel huileux est alors obtenu par refroidissement jusqu'à la température ambiante.

Le gel ainsi obtenu présente d'excellentes propriétés d'étalement et conduit à la formation d'un film très doux et protecteur.

### EXEMPLE 2 : Fond de teint

| *Phase A :* | |
|---|---|
| - Huile de silicone commercialisée sous la dénomination de "DC 556" par la Société Dow Corning | 14 g |
| - Microsphères creuses en matériau thermoplastique commercialisées sous la dénomination de "Expancel 551 DE" par la Société Casco-Nobel | 1,5 g |

| *Phase B :* | |
|---|---|
| - Homopolymère d'éthylène commercialisé sous la dénomination de "PEW 1555" par la Société Langer | 7 g |
| - Cire microcristalline | 4 g |

| *Phase C :* | |
|---|---|
| - Palmitate d'éthyl-2 hexyle | 19 g |
| - Isoparaffine hydrogénée | 14 g |
| - Lanolate d'isopropyle | 9.3 g |
| - Propyl paraben | 0.2 g |

| *Phase D :* | |
|---|---|
| - Oxydes de fer | 3 g |
| - Dioxyde de titane | 13 g |
| - Oxyde de zinc | 3 g |
| - Talc | 12 g |

On mélange les constituants des phases B et C puis chauffe à une température d'environ 107°C. Après fusion de tous les constituants, on homogénéise le mélange obtenu puis le refroidit à une température d'environ 90°C. On ajoute alors la phase A dont les constituants ont été préalablement mélangés à température ambiante, puis ajoute enfin les constituants de la phase D. Après homogénéisation, le mélange ainsi obtenu est coulé à chaud dans des coupelles.

Après refroidissement à la température ambiante, on obtient un fond de teint présentant d'excellentes propriétés d'étalement sur la peau ainsi qu'une très bonne tenue.

### EXEMPLE 3 : Rouge à lèvres

| *Phase A :* | |
|---|---|
| - Huile de silicone commercialisée sous la dénomination de "DC 556" par la Société Dow Corning | 23 g |

| *Phase B :* | |
|---|---|
| - Homopotymère d'éthylène commercialisé sous la dénomination de "PEW 1555" par la Société Langer | 7 g |
| - Cire microcristalline | 7 g |
| - Lanoline | 7 g |

| *Phase C :* | |
|---|---|
| - Huile de ricin | 22 g |
| - Huile de sésame | 22 g |

| *Phase D :* | |
|---|---|
| - Pigments | 12 g |

On mélange les constituants des phases B et C par chauffage à une température d'environ 107°C. Après fusion des cires, on homogénéise le mélange puis le refroidit à une température d'environ 95°C. On ajoute alors la phase A puis la phase D. Après homogénéisation du mélange, celui-ci est coulé dans des alvéoles en forme de bâton.

Après refroidissement, on obtient un rouge à lèvres s'appliquant très facilement sur les lèvres et présentant une très grande douceur. En outre, celui-ci possède une excellente tenue, c'est-à-dire une excellente résistance à l'usage et ne file pas.

### EXEMPLE 4 : Rouge à lèvres

| *Phase A :* | |
|---|---|
| - Huile de silicone commercialisée sous la dénomination de "Silbione 70633 V 30" par la Société Rhône Poulenc | 10 g |
| - Huile de jojoba | 25 g |
| - Huile de sésame | 27g |

| *Phase B :* | |
|---|---|
| - Homopolymère d'éthylène commercialisé sous la dénomination de "Polywax 500" par la Société Bareco | 20 g |
| - Lanoline | 6,5 g |

| *Phase C :* | |
|---|---|
| - Pigments | 11,5 g |

### EXEMPLE 5 : Rouge à lèvres

| *Phase A :* | |
|---|---|
| - Huile de silicone commercialisée sous la dénomination de "Silbione 70633 V 30" par la Société Phône Poulenc | 10 g |
| - Huile de jojoba | 25 g |
| - Huile de sésame | 27g |

| *Phase B :* | |
|---|---|
| - Copolymère d'éthylène et de propylène commercialisé sous la dénomination de "Petrolite GP-7" par la Société Bareco | 20 g |
| - Lanoline | 6,5 g |

| *Phase C :* | |
|---|---|
| - Pigments | 11,5 g |

Les rouges à lèvres des Exemples 4 et 5 sont préparés de manière similaire à celle de l'Exemple 3.

## Revendications

1. Procédé de préparation d'une composition cosmétique ou dermatologique anhydre ayant une phase grasse contenant l'association d'une huile de silicone et d'une cire, dans lequel, afin d'obtenir une phase grasse homogène, on mélange à chaud:
i) de 5 à 97 % en poids par rapport au poids total de ladite phase grasse, d'au moins une huile de silicone répondant à la formule suivante : 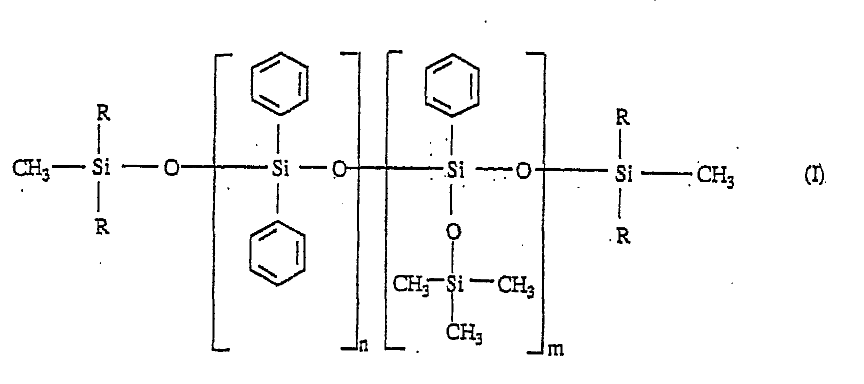 dans laquelle :
R représente un radical alkyle comportant 1 à 30 atomes de carbone, un radical aryle ou aralkyle,
n représente un nombre entier compris entre 0 et 100, et
m représente un nombre entier compris entre 0 et 100,
sous réserve que la somme n + m soit comprise entre 1 et 100, et
ii) de 3 à 50 % en poids par rapport au poids total de ladite phase grasse, d'une cire ayant un point de fusion compris entre 50 et 135°C, et étant constituée d'au moins un polymère de poids moléculaire compris entre 200 et 1.500 choisi parmi les homopolymères d'éthylène et les copolymères d'éthylène et d'un monomère répondant à la formule:
CH₂= CH-R' (II)
dans laquelle:
R' représente un radical alkyle ayant de 1 à 30 atomes de carbone, un radical aryle ou aralkyle.

2. Procédé selon la revendication 1, **caractérisé par le fait que** ladite phase grasse est présente en une proportion comprise entre 3 et 100 % en poids par rapport au poids total de la composition.

3. Procédé selon l'une quelconque des revendications précédentes, **caractérisé par le fait que** ladite huile de silicone présente une viscosité comprise entre 5 · 10⁻⁶ et 500 · 10⁻⁶ m²/s (5 et 500 cSt).

4. Procédé selon l'une quelconque des revendications précédentes, **caractérisé par le fait que** ladite huile de silicone est présente en une proportion comprise entre 10 et 90 % en poids par rapport au poids total de la phase grasse.

5. Procédé selon l'une quelconque des revendications précédentes, **caractérisé par le fait que** ladite cire est choisie parmi les homopolymères d'éthylène, les copolymères éthylène-propylène et les copolymères éthylène-hexène.

6. Procédé selon l'une quelconque des revendications précédentes, **caractérisé par le fait que** ladite cire est présente en une proportion comprise entre 5 et 30% en poids par rapport au poids total de la phase grasse.

7. Procédé selon l'une quelconque des revendications précédentes, **caractérisé par le fait que** ladite phase grasse comprend un mélange constitué (i) de 5 à 90% en poids de ladite huile de silicone par rapport au poids total de ladite phase grasse, et (ii) d'un homopolymère d'éthylène, ladite composition se présentant sous forme d'un produit de maquillage choisi parmi les fonds de teint et les rouges à lèvres.

8. Procédé selon l'une quelconque des revendications précédentes, **caractérisé par le fait que** ladite phase grasse comprend en outre au moins un additif ou corps gras choisi parmi une huile et/ou une cire.

9. Procédé selon la revendication 8, **caractérisé par le fait que** ledit additif ou corps gras est présent en une proportion comprise entre 0,5 et 92 % en poids par rapport au poids total de la phase grasse.

10. Procédé selon la revendication 9, **caractérisé par le fait que** ladite cire est présente en une proportion inférieure ou égale à celle de la cire d'homo- ou de copolymère d'éthylène telle que définie à la revendication 1.

11. Procédé selon l'une quelconque des revendications précédentes, **caractérisé par** la fait que ladite phase grasse comprend en outre un agent gélifiant huileux.

12. Procédé selon l'une quelconque des revendications précédentes, **caractérisé par le fait que** ladite composition comprend en outre des charges en une proportion comprise entre 0,5 et 97 %.

13. Procédé selon l'une quelconque des revendications précédentes, **caractérisé par le fait que** ladite composition comprend en outre des pigments.

14. Procédé selon l'une quelconque des revendications précédentes, **caractérisé par le fait que** ladite composition comprend en outre un additif lipophile choisi parmi les agents tensio-actifs, les filtres, les vitamines, les hormones, les agents anti-oxydants, les conservateurs, les colorants, les parfums et leurs mélanges.

## Claims

1. Process for preparing an anhydrous cosmetic or dermatological composition having a fatty phase comprising the combination of a silicone oil and a wax, wherein to obtain a homogeneous fatty phase the following ingredients are mixed in the hot state:
i) from 5 to 97% by weight, relative to the total weight of the said fatty phase, of at least one silicone oil corresponding to the following formula: 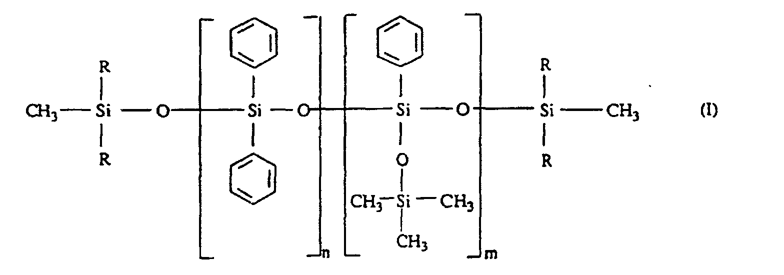 in which:
R represents an alkyl radical containing 1 to 30 carbon atoms, an aryl radical or an aralkyl radical,
n represents an integer between 0 and 100, and
m represents an integer between 0 and 100,
with the proviso that the sum n + m is between 1 and 100, and
ii) from 3 to 50% by weight, relative to the total weight of the said fatty phase, of a wax having a melting point of between 50 and 135°C, and consisting of at least one polymer with a molecular weight of between 200 and 1,500, chosen from ethylene homopolymers and copolymers of ethylene and of a monomer corresponding to the formula:
CH₂=CH-R' (II)
in which:
R' represents an alkyl radical having from 1 to 30 carbon atoms, an aryl radical or an aralkyl radical.

2. Process according to Claim 1, **characterized in that** the said fatty phase is present in a proportion of between 3 and 100% by weight relative to the total weight of the composition.

3. Process according to either of the preceding claims, **characterized in that** the said silicone oil has a viscosity of between 5 × 10⁻⁶ and 500 × 10⁻⁶ m²/s (5 and 500 cSt).

4. Process according to any one of the preceding claims, **characterized in that** the said silicone oil is present in a proportion of between 10 and 90% by weight relative to the total weight of the fatty phase.

5. Process according to any one of the preceding claims, **characterized in that** the said wax is chosen from ethylene homopolymers, ethylene/propylene copolymers and ethylene/hexene copolymers.

6. Process according to any one of the preceding claims, **characterized in that** the said wax is present in a proportion of between 5 and 30% by weight relative to the total weight of the fatty phase.

7. Process according to any one of the preceding claims, **characterized in that** the said fatty phase comprises a mixture consisting of (i) from 5 to 90% by weight of the said silicone oil relative to the total weight of the said fatty phase, and (ii) an ethylene homopolymer, the said composition being in the form of a make-up product chosen from foundations and lipsticks.

8. Process according to any one of the preceding claims, **characterized in that** the said fatty phase also comprises at least one additive or fatty substance chosen from an oil and/or a wax.

9. Process according to Claim 8, **characterized in that** the said additive or fatty substance is present in a proportion of between 0.5 and 92% by weight relative to the total weight of the fatty phase.

10. Process according to Claim 9, **characterized in that** the said wax is present in a proportion of less than or equal to that of the wax of ethylene homo- or copolymer as defined in Claim 1.

11. Process according to any one of the preceding claims, **characterized in that** the said fatty phase also comprises an oily gelling agent.

12. Process according to any one of the preceding claims, **characterized in that** the said composition also comprises fillers in a proportion of between 0.5 and 97%.

13. Process according to any one of the preceding claims, **characterized in that** the said composition also comprises pigments.

14. Process according to any one of the preceding claims, **characterized in that** the said composition also comprises a lipophilic additive chosen from surfactants, screening agents, vitamins, hormones, antioxidants, preserving agents, dyes, fragrances and mixtures thereof.

## Patentansprüche

1. Verfahren zur Herstellung einer kosmetischen oder dermatologischen, wasserfreien Zubereitung mit einer Fettphase, enthaltend eine Assoziation bzw. Verbindung eines Silikonöls und eines Wachses, worin man zum Erhalt einer homogenen Fettphase im Warmen mischt:
i) 5 bis 97 Gew.-% in Bezug auf das Gesamtgewicht der Fettphase mindestens eines Silikonöls der folgenden Formel: 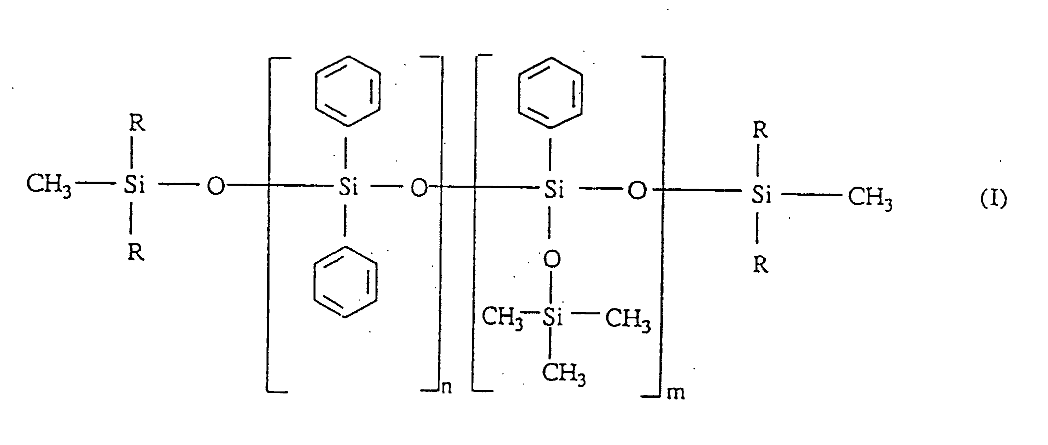 worin
R eine Alkylrest mit 1 bis 30 Kohlenstoffatomen, eine Aryl- oder Aralkylrest bedeutet,
n eine ganze Zahl zwischen 0 und 100 sowie
m eine ganze Zahl zwischen 0 und 100 darstellen, und Zwar
mit der Maßgabe, dass die Summe aus n + m zwischen 1 und 100 beträgt, sowie
ii) 3 bis 50 Gew.-% eines Wachses mit einem Schmelzpunkt zwischen 50 und 135°C, bezogen auf das Gesamtgewicht der Fettphase, das aus mindestens einem Polymeren mit einem Molekulargewicht zwischen 200 und 1500 besteht, das aus Ethylenhomopolymeren und Copolymeren von Ethylen mit einem Monomer ausgewählt ist, das der folgenden Formel entspricht:
CH₂=CH-R' (II)
worin
R' einen Alkylrest mit 1 bis 30 Kohlenstoffatomen, einen Aryl- oder Aralkylrest deutet.

2. Verfahren nach Anspruch 1, **dadurch gekennzeichnet, dass** die Fettphase in einem Mengenverhältnis zwischen 3 und 100 Gew.-% in Bezug auf das Gesamtgewicht der Zusammensetzung vorhanden ist.

3. Verfahren nach einem der vorstehenden Ansprüche, **dadurch gekennzeichnet, dass** das Silikonöl eine Viskosität aufweist, die zwischen 5 • 10⁻⁶ und 500 • 10⁻⁶ m²/s (5 und 500 cSt) beträgt.

4. Verfahren nach einem der vorstehenden Ansprüche, **dadurch gekennzeichnet, dass** das Silikonöl in einem Mengenverhältnis vorhanden ist, das zwischen 10 und 90 Gew.-% in Bezug auf das Gesamtgewicht der Fettphase beträgt.

5. Verfahren nach einem der vorstehenden Ansprüche, **dadurch gekennzeichnet, dass** das Wachs aus Ethylenhomopolymeren, Ethylen/Propylen-Copolymeren und Ethylen/Hexen-Copolymeren ausgewählt ist.

6. Verfahren nach einem der vorstehenden Ansprüche, **dadurch gekennzeichnet, dass** das Wachs in einem Mengenverhältnis zwischen 5 und 30 Gew.-% in Bezug auf das Gesamtgewicht der Fettphase vorhanden ist.

7. Verfahren nach einem der vorstehenden Ansprüche, **dadurch gekennzeichnet, dass** die Fettphase ein Gemisch umfasst, das aus
(i) 5 bis 90 Gew.-% des Silikonöls in Bezug auf das Gesamtgewicht der Fettphase sowie
(ii) einem Ethylenhomopolymer besteht, wobei die Zubereitung in Form eines Schminkproduktes vorliegt, das aus Teintgrundlagen und Lippenstift(Lippenrouge)-Zubereitung ausgewählt ist.

8. verfahren nach einem der vorstehenden Ansprüche, **dadurch gekennzeichnet, dass** die Fettphase zusätzlich noch mindestens einen Hilfsstoff oder einen Fettkörper umfasst, der aus einem Öl und/oder einem Wachs ausgewählt ist.

9. Verfahren nach Anspruch 8, **dadurch gekennzeichnet, dass** dieser Hilfsstoff oder Fettkörper in einem Mengenverhältnis vorhanden ist, das zwischen 0,5 und 92 Gew.-% in Bezug auf das Gesamtgewicht der Fettphase beträgt.

10. Verfahren nach Anspruch 9, **dadurch gekennzeichnet, dass** dieses Wachs in einem Mengenverhältnis von weniger oder gleich demjenigen des Wachses aus dem Ethylenhomo- oder Copolymer gemäß Anspruch 1 vorhanden ist.

11. Verfahren nach einen der vorstehenden Ansprüche, **dadurch gekennzeichnet, dass** die Fettphase zusätzlich noch ein öliges Geliermittel umfasst.

12. Verfahren nach einem der vorstehenden Ansprüche, **dadurch gekennzeichnet, dass** die Zusammensetzung zusätzlich noch Trägerstoffe bzw. Zuschläge in einem Mengenverhältnis aufweist, das zwischen 0,5 und 97 % beträgt.

13. Verfahren nach einem der vorstehenden Ansprüche, **dadurch gekennzeichnet, dass** deren Zusammensetzung zusätzlich noch Pigmente umfasst.

14. Verfahren nach einem der vorstehenden Ansprüche, **dadurch gekennzeichnet, dass** die Zusammensetzung zusätzlich noch einen lipophilen Hilfsstoff umfasst, der aus Tensiden, Filtersubstanzen, Vitaminen, Hormonen, Antioxidantien, Konservierungsmitteln, Farbstoffen, Duftstoffen sowie deren Gemischen ausgewählt ist.
